# EUROPEAN PATENT SPECIFICATION

(11) **EP 0 317 004 B1**
(45) Date of publication and mention of the grant of the patent: **02.03.1994**
(21) Application number: 88202526.5
(22) Date of filing: 10.11.1988
(51) Int. Cl.: C07D 307/60, C07C 57/13, C07C 51/567

(54) **Process for the preparation of a substituted succinic anhydride**
Verfahren zur Herstellung von substituiertem Bernsteinsäureanhydrid
Procédé de préparation d'anhydride succinique substitué

(30) Priority: 13.11.1987 US 120255
(43) Date of publication of application: 24.05.1989
(73) Proprietor: SHELL INTERNATIONALE RESEARCH MAATSCHAPPIJ B.V., 2596 HR Den Haag (NL)
(72) Inventor: Fried, Herbert Elliott, Houston, TX 77042 (US); Edwards, Charles Lee, Houston, TX 77077 (US)

(56) References cited:
- DE-A- 3 644 222
- US-A- 4 085 143

## Description

This invention relates to a process for the preparation of a substituted succinic anhydride by reacting an olefinically unsaturated compound with maleic anhydride in the presence of a catalytic amount of an additive which inhibits side-reactions.

Reactions of olefinically unsaturated compounds with maleic anhydride at elevated temperatures give the corresponding adducts in accordance with the following equation: 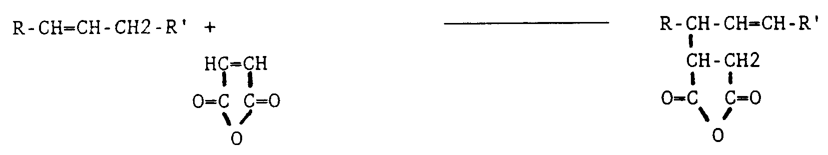
(R and R′ represent independently hydrogen atoms or optionally substituted hydrocarbyl groups). However, reactions of this type require very long reaction times even at elevated temperatures. In addition, under conventional temperature conditions (200-300°C), darkening of the product and formation of black solids are typically observed. These problems are believed to arise from secondary reactions involving maleic anhydride for example, polymerization and decarboxylation. Thus, stabilization of maleic anhydride for these secondary reactions and acceleration of the addition reaction is desirable.

It is known that the reaction of the olefinically unsaturated hydrocarbons with maleic anhydride can be carried out in the presence of a catalytically effective amount of an additive in order to accelerate the reaction. For example, in conventional processes, the reaction times are reduced to a satisfactory level by carrying out the addition reactions in the presence of small amounts of substances such as furan derivatives (US-4,388,471), iodine (GB-1,356,802), bromine (GB-1,480,453), an α-bromodialkylketone (US-3,953,475 and US-3,954,812), hydrogen chloride or calcium bromide (US-3,935,249), a hydantoin derivative (US-3,927,041), p-toluenesulfonic acid (US-3,855,251), a nickel salt (GB-2,081,274) or a bromophenol (US-4,278,604).

In these processes, however, the degree of conversion of the olefin is frequently low. In addition, where halogen compounds are used, extra precautions have to be taken due to the toxicity of the reaction mixture. Many of these conventional processes also have the disadvantages of producing discoloration and formation of solids during the reaction which contaminate the kettle walls or, in more adverse cases, the reaction product. An even more disadvantageous feature is the formation of resin-like residues which render the product useless if it cannot be purified by distillation or filtration.

US-A-4,086,251 discloses a process for the preparation of a substituted succinic anhydride by the reaction of a polypropene or a polybutene with maleic acid in the presence of the additive to suppress the formation of tar and side products. Examples of such additives include chlorinated or brominated aliphatic hydrocarbons, chlorine and/or bromine-containing derivatives of carboxylic or sulphonic acids, chlorinated and/or brominated intramolecular anhydrides of aliphatic carboxylic acids and several other chlorine or bromine-containing organic or inorganic compounds.

It is known to use certain metals or organometallic compounds to prevent these side-reactions. Thus it has been proposed in US-4,396,774 to use an alkyl aluminium halide. However, these halogenated compounds necessitate the presence of halogenated solvents, and may pose environmental problems. It is proposed in US-4,599,433 to carry out the above adduction reaction in the presence of an alkoxide of titanium, zirconium, vanadium or aluminium. Titanium (IV) n-butoxide is the only alkoxide exemplified and is rather expensive.

It is the aim of the present invention to provide a process for the preparation of substituted succinic anhydrides which can be carried out using a side-reaction-inhibiting additive which does not contain halogen atoms and is inexpensive, and which process does not necessitate the use of a solvent, so that discolouration and resin-like residue formation is avoided.

It has now been found that this objective is met by using small amounts of alkoxides of zinc or dialkyl zinc as the additive, which reduce the formation of black solids and improve product color for the reaction of maleic anhydride and olefinically unsaturated compounds to produce substituted succinic anhydrides. The presence of these additives permits the reaction to be conducted at higher temperatures which decreases residence times and allows complete consumption of maleic anhydride to avoid plugging or recycle.

Accordingly, the present invention relates to a process for the preparation of a substituted succinic anhydride by reacting an olefinically unsaturated compound with maleic anhydride in the presence of a catalytic amount of an additive which inhibits side-reactions, characterized in that an alkoxide of zinc or dialkyl zinc is present as the additive.

Suitable olefinically unsaturated compounds for the process of the invention are all compounds which possess terminal double bonds or double bonds within a chain and have a molecular weight in the range of from about 100 to about 3000, and mixtures of these compounds. Though hydrocarbons are preferred compounds with functional groups, e.g. acrylate esters, are suitable too.

The term "olefinically unsaturated hydrocarbons" as used herein, refers to monomeric, oligomeric and polymeric C₇-C₂₀₀ alkenes whose chains may or may not be branched. The olefinic unsaturated hydrocarbons which can be subjected to the addition reaction include, for example, tetradecene-1, oct-1-ene, 2,4,4-trimethylpent-2-ene, 2-methyl-5-propylhex-1-ene, 3-cyclohexyl-bute-1-ene and the oligomers of C₂-C₂₀ olefins, for example the oligomers of ethylene, propylene, but-1-ene, isobutene, hex-1-ene, oct-1-ene, and the like, and the polyisobutenes where the molecular weight is from about 350 to about 3000, and diisobutene. Preferred olefinically unsaturated hydrocarbons are C₁₄-C₂₀ linear internal or branched olefins and alpha olefins.

In the reaction of the olefinically unsaturated compounds with maleic anhydride, the molar ratio of maleic anhydride to olefin, i.e., the proportions of substances based on the number of moles of components, is typically from 0.4:1 to 5.0:1, preferably from 0.65:1 to 1.2:1, in particular from 0.8:1 to 1:1. A process in which equal molar amounts of olefinic compound and maleic anhydride can be used is particularly preferred.

To avoid side reactions during the addition reaction of maleic anhydride, the reaction is carried out in the presence of from 1 to 5000, preferably from 5 to 1000, ppm by weight, based on the weight of the reactants, of zinc alkoxide or dialkyl zinc additive. The principle side reactions are believed to be the formation of poly(maleic anhydride), which is obtained as solid residue, or poly(maleic anhydride) with an olefinic component from free radical copolymerization of the olefin and the maleic anhydride. The addition reaction with formation of the corresponding succinic anhydrides takes place at from 160°C to 260°C, preferably from 230°C to 245°C. The reaction is preferably carried out in an agitated reactor either in the presence or in the absence of a solvent, although no solvent is typically required. The reaction times are typically from 1 hour to 20 hours, preferably from 4 hours to 10 hours and especially from 3.5 hours to 5 hours. In a preferred embodiment, the reaction is carried out in an essentially oxygen-free atmosphere in an autoclave in the presence of an inert gas. A nitrogen or argon atmosphere is preferably used as the inert atmosphere. When the reaction is complete, the autoclave is left to cool and the reaction mass is preferably worked up by distillation. As far as possible, the reactants should be anhydrous.

In the present process the additives used are compounds of zinc having the formula:

A-(O)ₙ-Zn-(o)ₙ-A′

where A and A′ each individually represent an alkyl group suitably from 1 to 20 carbon atoms, preferably from 1 to 10 carbon atoms and more preferably from 1 to 4 carbon atoms, and n is 0 or 1. The values of both integers "n" need not be equal. Particularly suitable alkoxides of zinc include diethoxy zinc, di-n-butoxy zinc, diisopropoxy zinc, dimethoxy zinc, dihexadecyloxy zinc, dioctyloxy zinc, didodecyloxy zinc and the like. The stated alkoxides are in the solid state and are used in this form in the addition reaction. The alkoxides of zinc utilized in the present process are typically prepared by reacting a dialkyl zinc such as diethyl zinc with the corresponding primary, secondary or tertiary alcohol. Dialkyl zinc compounds particularly suitable as additives in the instant invention include compounds having from C₁ to C₂₀ carbon atoms on each alkyl moiety such as, for example, dimethyl zinc, diethyl zinc, dibutyl zinc, diisopropyl zinc and the like.

One advantage obtained when the invention is used in the absence of solvents is the fact that no toxic e.g. halogen-containing products are formed. In addition, the use of small amounts of alkoxides of zinc dramatically reduces sludge-make and improves the product colour.

The resulting products having molecular weights in the range of from about 200 to about 350 are used for the preparation of anticorrosive agents for aqueous or organic systems. The resulting olefinic-succinic anhydrides having molecular weights in the range of from about 250 to about 3000 can be converted in simple manner to compounds which are suitable as oil additives such as for example, lubricant additives.

The instant invention will now be described by the use of the following examples. The results are presented in the Table.

### Example 1

37.0 Grams of tetradecene-1 having a molecular weight of 196 and 18.0 grams of maleic anhydride (a maleic anhydride to olefin ratio of 1:1) were reacted in a Fischer-Porter bottle in the presence of 0.02 grams of diethoxy zinc while being stirred with a magnetic stirrer. The reaction mixture was then heated to 245°C for 4 hours.

### Example 2

The procedure of Example 1 was repeated except that di-n-butoxy zinc was used in place of diethoxy zinc as additive.

### Example 3

37.0 Grams of a C₁₅-C₂₀ internal olefin mixture having an average molecular weight of 239 was sparged with nitrogen for about 16 hours and then reacted with 12.1 grams of maleic anhydride (a maleic anhydride to olefin ratio of 0.8:1) in a Fischer-Porter bottle in the presence of 0.02 grams of di-n-butoxy zinc while being stirred with a magnetic stirrer. The reaction mixture was then heated to 230°C for 20 hours.

### Comparative Example A

The procedure of Example 1 was repeated except that no additive was used.

### Comparative Example B

The procedure of Example 3 was repeated except that no additive was used.

**TABLE**

| Example | Reaction time (hr) | Temperature (°C) | Olefin (%w)^{a)} | Substituted Anhydride (%w)^{a)} | Klett colour |
|---|---|---|---|---|---|
| 1 | 4 | 245 | alpha (23.1) | 76.9 | 202 |
| 2 | 4 | 245 | alpha (24) | 76 | 268 |
| 3 | 20 | 230 | internal (25.5) | 74.5 | 248 |
| A | 4 | 245 | alpha (26.5) | 73.4 | 560^{b)} |
| B | 20 | 230 | internal (38) | 62.0 | 286^{b)} |

| | | | | | |
|---|---|---|---|---|---|
| a) Determined by gas liquid chromatography. The value for substituted anhydride includes both the 1:1 and the 2:1 maleic anhydride/olefin adducts (the latter comprising less than 6%). | | | | | |
| b) Significant amounts of black solids observed. | | | | | |

As can be seen from the Table, the presence of alkoxides of zinc as additives in the reaction of olefins and maleic anhydride results in a product having reduced formation of side reactions of soluble and/or insoluble contaminants. In addition, as evidenced by these examples, the use of these additives results in reduction of soluble by-products and improved product colour.

## Claims

1. Process for the preparation of a substituted succinic anhydride by reacting an olefinically unsaturated compound with maleic anhydride in the presence of a catalytic amount of an additive which inhibits side-reactions, characterized in that an alkoxide of zinc or dialkyl zinc is present as the additive.

2. A process as claimed in claim 1 wherein the additive has the formula:
A-(O)ₙ-Zn-(O)ₙ-A′ wherein A and A′ each individually represent an alkyl group having from 1 to 20 carbon atoms, and each n independently is 0 or 1.

3. A process as claimed in claim 2 wherein A and A′ each individually represent an alkyl group having from 1 to 10 carbon atoms, and n = 1.

4. A process as claimed in claim 3, wherein the additive is selected from the group consisting of diethoxy zinc, di-n-butoxy zinc, diisopropoxy zinc, dimethoxy zinc, and dioctyloxy zinc.

5. A process as claimed in claim 4, wherein the additive is diethoxy zinc or di-n-butoxy zinc.

6. A process as claimed in any of claims 1-5, wherein the amount of additive is in the range of 1 to 5000 ppm by weight, based on total weight of reactants.

7. A process as claimed in claim 6, wherein the amount of additive is in the range of 5 to 1000 ppm by weight based on total weight of reactants.

8. A process as claimed in any of claims 1-7, wherein an alkenyl substituted succinic anhydride is prepared by reacting a C₇₋₂₀₀ olefin with maleic anhydride.

9. A process as claimed in claim 8, wherein the olefin is a C₁₄-C₂₀ linear or branched internal or alpha-olefin, or a mixture thereof.

## Patentansprüche

1. Verfahren zur Herstellung eines substituierten Bernsteinsäureanhydrids durch Umsetzen einer olefinisch ungesättigten Verbindung mit Maleinsäureanhydrid in Gegenwart einer katalytischen Menge eines Additivs, welches Nebenreaktionen verhindert, dadurch gekennzeichnet daß ein Alkoxid von Zink oder ein Dialkylzink als Additiv vorliegt.

2. Ein Verfahren wie in Anspruch 1 beansprucht, in welchem das Additiv die Formel
A-(O)ₙ-Zn-(O)ₙ-A' hat, wobei A und A' jeweils unabhängig eine Alkylgruppe mit 1 bis 20 Kohlenstoffatomen darstellen und jedes n jeweils unabhängig den Wert O oder 1 hat.

3. Ein Verfahren wie in Anspruch 2 beansprucht, in welchem A und A' jeweils unabhängig eine Alkylgruppe mit 1 bis 10 Kohlenstoffatomen darstellt und n den Wert 1 hat.

4. Ein Verfahren wie in Anspruch 3 beansprucht, in welchem das Additiv ausgewählt ist aus der Gruppe, bestehend aus Diethoxyzink, Di-n-but-oxyzink, Diisopropoxyzink, Dimethoxyzink und Dioctyloxyzink.

5. Ein Verfahren wie in Anspruch 4 beansprucht, in welchem das Additiv Diethoxyzink oder Di-n-butoxyzink ist.

6. Ein Verfahren wie in irgendeinem der Ansprüche 1 bis 5 beansprucht, in welchem die Menge an Additiv im Bereich von 1 bis 5000 Gewichtsteilen pro Million, bezogen auf das Gesamtgewicht der Reaktionsteilnehmer, liegt.

7. Ein Verfahren wie in Anspruch 6 beansprucht, in welchem die Menge an Additiv im Bereich von 5 bis 1000 Gewichtsteilen pro Million, bezogen auf das Gesamtgewicht der Reaktionsteilnehmer, liegt.

8. Ein Verfahren wie in irgendeinem der Ansprüche 1 bis 7 beansprucht, in welchem ein alkenyl-substituiertes Bernsteinsäureanhydrid durch Umsetzung eines C₇₋₂₀₀-Olefins mit Maleinsäureanhydrid hergestellt wird.

9. Ein Verfahren wie in Anspruch 8 beansprucht, in welchem das Olefin ein lineares oder ein verzweigtes inneres oder ein alpha-Olefin mit 14 bis 20 Kohlenstoffatomen oder eine Mischung davon ist.

## Revendications

1. Procédé pour la préparation d'anhydride succinique substitué par réaction d'un composé non saturé de manière oléfinique avec de l'anhydride maléique en présence d'une quantité ayant un effet catalytique d'un additif qui inhibe les réactions secondaires, caractérisé en ce que de l'alkoxyde de zinc ou du dialkyl zinc se trouve présent en tant qu'additif.

2. Procédé tel que revendiqué dans la revendication 1, dans lequel l'additif possède la formule
A-(O)ₙ-Zn-(O)ₙ-A' dans laquelle A et A' représentent chacun individuellement un groupe alkyle ayant de 1 à 20 atomes de carbone et chaque n valant indépendamment 0 ou 1.

3. Procédé tel que revendiqué dans la revendication 2, dans lequel A et A' représentent chacun individuellement un groupe alkyle ayant de 1 à 10 atomes de carbone, et n = 1.

4. Procédé tel que revendiqué dans la revendication 3, dans lequel l'additif est choisi dans le groupe composé du diéthoxy zinc, du di-n-butoxy zinc, du diisopropoxy zinc, du diméthoxy zinc, et du dioctyloxy zinc.

5. Procédé tel que revendiqué dans la revendication 4, dans lequel l'additif est du dioéthoxy zinc ou du di-n-butoxy zinc.

6. Procédé tel que revendiqué dans l'une quelconque des revendications 1 à 5, dans lequel la quantité d'additif est comprise entre 1 et 5000 ppm en poids, par rapport au poids total des réactifs.

7. Procédé tel que revendiqué dans la revendication 6, dans lequel la quantité d'additif est comprise entre 5 et 1000 ppm en poids par rapport au poids total des réactifs.

8. Procédé tel que revendiqué dans l'une quelconque des revendications 1 à 7, dans lequel un anhydride succinique alkényl substitué est préparé en faisant réagir une oléfine en C₇ à C₂₀₀ avec de l'anhydride maléique.

9. Procédé tel que revendiqué dans la revendication 8, dans lequel l'oléfine est une oléfine interne linéaire ou ramifiée ou une alpha-oléfine en C₁₄ à C₂₀, ou un mélange de celles-ci.
